# EUROPÄISCHE PATENTANMELDUNG

(11) **EP 1 795 585 A1**
(43) Veröffentlichungstag der Anmeldung: **13.06.2007**
(21) Anmeldenummer: 05027051.1
(22) Anmeldetag: 10.12.2005
(51) Int. Cl.: C12M 1/113, C12M 1/107, C12M 1/16

(54) **Einstufiges Betriebsverfahren für eine Durchfluss-Nassfermentations-Biogasanlage**

(71) Anmelder: U.T.S. Umwelt-Technik-Süd GmbH, 84419 Obertaufkirchen (DE)
(72) Erfinder: Bürger, Adam, 83527 Haag/Obb. (DE)
(74) Vertreter: Neubauer, Hans-Jürgen

(57) **Zusammenfassung**

Die Erfindung betrifft ein Betriebsverfahren zur Nassfermentation in einer einstufigen, eingefahrenen und im Durchflussverfahren arbeitenden Biogasanlage (1), mit wenigstens einem ein Substrat mit bestimmten Trockensubstanzgehalt enthaltenden Fermenter (2, 2') mit einer vorgeschalteten Zudosiereinrichtung (3) für vergärbare Stoffe und mit einem nachgeordneten Separator (6; 21) mit dem eine zumindest teilweise Trennung eines aus dem Fermenter (2, 2') abgezogenen Substrats in einen Substrat-Feststoffanteil (7) und einen Substrat-Flüssigkeitsanteil (9) erfolgt. Erfindungsgemäß wird mit der Zudosiereinrichtung (3) eine vorgegebene Menge an vergärbaren Stoffen kontinuierlich oder chargenweise quasi kontinuierlich in den Fermenter (2, 2') eingebracht und eine entsprechende Menge an Substrat dem Fermenter (2, 2') entnommen und zumindest teilweise dem Separator (6; 21) zugeführt. Zumindest ein Teil des durch den Separator (6; 21) separierten Substrat-Feststoffanteils (7) wird unmittelbar in den Fermenter (2, 2') und/oder über die Zudosiereinrichtung (3) in den Fermenter zurückgeführt. Dadurch wird das in den vergärbaren Stoffen enthaltene Energiepotential mehr genutzt.

## Beschreibung

Die Erfindung betrifft ein Betriebsverfahren zur Nassfermentation in einer einstufigen, eingefahrenen und im Durchflussverfahren arbeitenden Biogasanlage nach dem Oberbegriff des Anspruchs 1.

Die Biogasgewinnung zur Energieerzeugung gewinnt vorrangig im landwirtschaftlichen Bereich zunehmend an Bedeutung. Die Gründe dafür sind im wesentlichen die Erschließung neuer Einkommensquellen in der Landwirtschaft durch eine staatlich geförderte regenerative und umweltverträgliche Energiegewinnung. Die dafür bekannten Biogasanlagen sind meist für einen kontinuierlichen einstufigen Betrieb ausgelegt, mit einem Fermenterbehälter, in dem in einem anaeroben Nassvergärungsprozess zur Gewinnung von Biogas Biomassen als anorganische Substrate vergoren werden. Je nach den Gegebenheiten sind einem solchen Fermenter ein Nachfermenter und ein Endlager sowie separate Gasspeicher für das erzeugte Biogas nachgeordnet. Zur Beschickung des Fermenterbehälters mit vergärbaren Stoffen ist diesem eine meist automatisierte Zudosiereinrichtung vorgeschaltet, wobei für flüssige Substratbestandteile Pumpen und für feste Biomassen Feststoff-Zudosiereinrichtungen mit Wägeeinrichtungen und Befüllschnecken oder Förderbändern verwendet werden. Zudem sind in den Behältern und Fermentern Rühreinrichtungen für eine gleichmäßige Verteilung der Substratbestandteile angebracht (EP 1 251 165 A1).

Die Erzeugung von Biogas erfolgt bei der anaeroben Nassvergärung im wesentlichen in vier Schritten, der Hydrolyse, der Versäuerungsphase (Acidogenese), der Essigsäurebildung (Acetogenese) und der Methanogenese.

Bei der dem erfindungsgemäßen Betriebsverfahren zugrundeliegenden einstufigen Biogasanlage laufen die vorstehenden Prozesse ohne räumliche Trennung gemeinsam und parallel ab. Ein solcher stabiler, eingefahrener und kontinuierlicher Prozessverlauf wird jedoch erst nach einer Einfahrphase erreicht, die nicht Gegenstand des vorliegenden Betriebsverfahrens ist.

Ein wesentlicher Aspekt der Biogasgewinnung ist die Nutzung zur Einspeisung in ein der Biogasanlage zugeordnetes Blockheizkraftwerk für eine wirtschaftlich lukrative Erzeugung von Elektroenergie, welche über staatlich festgelegte Vergütungssätze gefördert und unterstützt wird.

Der vorstehende, einstufige und kontinuierliche Betrieb zur Nassfermentation ist bei Verwendung des Durchflussverfahrens mit einem relativ einfachen und kostengünstigen Anlagenaufbau zu realisieren und durch Automatisierungsmöglichkeiten mit angemessenem Aufwand durchzuführen.

Dabei ist es für einen störungsfreien Gärprozess und Anlagenbetrieb wesentlich im Durchflussverfahren ein möglichst gleiches Füllniveau im Fermenter und ggf. in einem nachgeordneten Nachfermenter aufrecht zu erhalten. Dazu wird die durch eine kontinuierliche oder chargenweise, quasi kontinuierliche Beschickung mittels der Zudosiereinrichtung dem Fermenter zugeführte Beschickungsmenge als gleiche, zumindest teilweise entsprechend der Verweildauer vergorene Substratmenge dem Fermenter entnommen. Diese Entnahme erfolgt in bekannter Weise durch einen Überlauf oder durch einen Pumpvorgang in einen Nachfermenter oder in ein Endlager. Ohne diese Entnahme wäre der Fermenter durch die ständige Zudosierung in Kürze unzulässig überfüllt. In dieser bedingt durch das Durchlassverfahren jeweils zwangsläufig zu entnehmenden, wegen der begrenzten Verweildauer im Fermenter nicht voll vergorenen Substratmenge ist noch ein erhebliches Restpotential für eine weitere Vergärung und Biogasgewinnung enthalten. Wird diese Substratmenge unmittelbar einem Endlager zugeführt, bleibt dieses Restpotential ungenutzt. Wird diese Substratmenge in an sich bekannter Weise einem Nachfermenter zugeführt, ergibt sich die gleiche Situation lediglich mit reduziertem Restpotential nach dem Nachfermenter. In beiden Fällen wird nachteilig das noch im Substrat-Feststoffanteil des abgezogenen Substrats enthaltene Energiepotential und damit Biogaspotential nicht gentutzt.

Die hier verfahrensmäßig zugrundeliegenden Nassfermentation ist mit einem Trockensubstanzgehalt von maximal etwa 15% im Fermenter durchführbar, bei dem das Substrat für die vorstehende einfache Entnahme zum Füllniveauausgleich auch noch ausreichend fließ- und pumpfähig ist. Durch diese Entnahmeart wird dem Fermenter relativ viel Substrat-Flüssiganteil entnommen. Insbesondere bei einer Beschickung mit hohem Feststoffanteil oder bei einer ausschließlichen Feststoffbeschickung würde innerhalb kurzer Zeit der Trockensubstanzgehalt unzulässig hoch ansteigen. Um dies zu unterbinden, wird regelmäßig Flüssigkeit zur Verdünnung bei der Beschickung mit zugeführt.

Bei landwirtschaftlichen Betrieben mit Tierhaltung ist dies kein Problem, da anfallende Gülle als Verdünnungsflüssigkeit zusammen mit festen Biomassen ggf. nach einem Anmaischen in einer Anmaischgrube zugeführt werden kann.

Viele Iandwirtschaftliche Betriebe haben jedoch keine Tierhaltung, so dass dort keine Gülle zur Verfügung steht. Bei dort installierten Biogasanlagen wird zur Verdünnung üblicherweise Wasser, meist Wasser aus der Trinkwasserversorgung beigemischt. Dies ist einerseits unwirtschaftlich und zudem wenig umweltverträglich.

Es ist zudem allgemein bekannt, einem Fermenter einen Separator nachzuordnen, mit dem das für einen Niveauausgleich abgezogene Substrat zumindest teilweise in einen Substrat-Feststoffanteil und einen Substrat-Flüssigkeitsanteil separiert wird. Ziel einer solchen bekannten Fest-Flüssig-Trennung durch einen nachgeschalteten Separator ist es, aus dem abgezogenen Substrat einen hohen Substrat-Feststoffanteil auszuscheiden und diesen nicht mehr einem Endlager zuzuführen, um dort Schwimmschichten zu reduzieren und/oder dort Rührvorgänge zu reduzieren, sowie die Ausbringung eines flüssigeren Endlagerinhalts zu vereinfachen. Der separierte Substrat-Feststoffanteil wird somit aus dem Prozess herausgenommen und kompostiert oder als Dünger ausgebracht. Weiter kann der am Separator anfallende Substrat-Flüssigkeitsanteil als Efluent unspezifisch zum Teil wieder als Bestandteil einer Anmaischflüssigkeit in Verbindung mit Gülle und/oder Wasser zur Verdünnung dem Fermenter zugeführt werden.

Aufgabe der Erfindung ist es, ein Betriebsverfahren vorzuschlagen, mit dem der wirtschaftliche Betrieb und die Umweltverträglichkeit einer mit Nassfermentation im einstufigen Durchflussverfahren arbeitenden, eingefahrenen Biogasanlage verbessert werden.

Diese Aufgabe wird mit den Merkmalen des Anspruchs 1 gelöst.

Gemäß Anspruch 1 wird mit der Zudosiereinrichtung eine vorgegebene Menge an vergärbaren Stoffen kontinuierlich oder chargenweise quasi kontinuierlich in den Fermenter eingebracht und zur Aufrechterhaltung eines im wesentlichen gleichen Füllniveaus im Fermenter eine entsprechende Menge an Substrat aus dem Fermenter durch Verdrängung oder Pumpen entnommen, wobei dieses Substrat zumindest teilweise dem Separator zugeführt wird. Der hier betrachtete eingefahrene Betriebszustand der Biogasanlage umfasst auch den Betrieb einer im Fermenter installierten Rührtechnik, so dass die Betrachtungen hier insbesondere ein betriebsmäßig gerührtes und durchmischtes Substrat betreffen.

Zumindest ein Teil des durch den Separator separierten Substrat-Feststoffanteils wird unmittelbar in den Fermenter und/oder über die Zudosiereinrichtung in den Fermenter zurückgeführt.

Dem liegt die Erkenntnis zugrunde, dass beim vorliegenden Durchflussverfahrenbetrieb entsprechend der Zudosierung Substrat aus dem Fermenter abgezogen werden muss und in diesem Substrat zwangsläufig noch ein relativ hoher Anteil weiter vergärbarer Feststoffanteile enthalten ist. Dies ist auch dann der Fall, wenn Substrat aus einem dem Fermenter nachgeordneten Nachfermenter abgezogen wird. Ersichtlich kann das für eine Stabilisierung des Füllniveaus abgezogene Substrat nicht insgesamt in den Fermenter zurückgeführt werden, da dann das Füllniveau wegen der zusätzlichen Beschickungsmenge kontinuierlich steigen würde. Nach einer Fest-Flüssig-trennung nach dem Separator, lieget jedoch ein separierter Substrat-Feststoffanteil mit wesentlich kleinerem Volumen vor, mit einem hohen Anteil noch weiter vergärbarer Stoffe und damit noch nicht genutztem Energiepotential bzw. Biogaspotential. Dieser separierte Substrat-Feststoffanteil mit einem gegenüber dem abgezogenen Substrat reduzierten Volumen, kann daher ohne das Füllniveau zu erhöhen, dem Fermenter wieder zugegeben werden, wenn der separierte Substrat-Flüssigkeitsanteil nicht mehr oder nur eine relativ kleine Menge in den Fermenter zurückgeführt wird.

Damit wird durch die nochmalige Vergärung bzw. Weitervergärung des separierten Substrat-Feststoffanteils das Energiepotential der eingesetzten und zudosierten Biomassen mehr ausgenützt, was einerseits die Wirtschaftlichkeit erhöht und zudem durch die bessere Ausnutzung des möglichen Energiepotentials die Umweltverträglichkeit steigert. Zudem sind in den separierten Substrat-Feststoffanteilen auch bereits die geeigneten Bakterien enthalten, die bei einer Rückführung in den Fermenter die Weitervergärung begünstigen.

Eine vorteilhaft besonders hohe Energieausbeute wird erreicht, wenn nach Anspruch 2 das gesamte für eine Niveaustabilisierung entnommene Substrat dem Separator zugeführt wird und gemäß Anspruch 3 der gesamte durch den Separator daraus separierte Substrat-Feststoffanteil in den Fermenter zurückgeführt wird.

Grundsätzlich kann diese Rückführung des separierten Substrat-Feststoffanteils, insbesondere in die Zudosiereinrichtung von einer Person beispielsweise mit einem Frontlader ausgeführt werden. Eine Vereinfachung, Reduzierung des Arbeitsaufwands und Vergleichmäßigung der Beschickung und der Zusammensetzung des Beschickungsmaterials wird gemäß Anspruch 4 dadurch erreicht, dass der Substratfeststoffanteil vom Separator automatisiert durch Fördermittel kontinuierlich oder chargenweise der Zudosiereinrichtung zugeführt wird. Als Fördermittel können hier die in Verbindung mit Zudosiereinrichtungen bekannten Fördermittel, wie Förderbänder, Förderschnecken, Förderkolben etc. eingesetzt werden.

Als Separator können nach Anspruch 5 je nach den Anlagebedingungen und Betriebsbedingungen jeweils einer oder mehrere an sich bekannte Schrauben- bzw. Schnecken-Separatoren und/oder Zentrifugen-Separatoren und/oder Siebbandpressen verwendet werden, wobei jeder Separatortyp hinsichtlich der Effektivität der Separierung und des Betriebs und der Wartung unterschiedliche an sich bekannte Eigenschaften aufweist, die bei einer Entscheidung für einen bestimmten Separatortyp zu berücksichtigen sind.

Als Alternative oder zusätzliche Separatoranordnung wird nach Anspruch 6 ein Separator mit einem Separatorbehälter vorgeschlagen, in dem eine Schüttung aus Biofeststoffen als Feststofffilter eingebracht ist, über die das abgezogene Substrat geleitet wird. In der Schüttung wird dabei der Substrat-Feststoffanteil weitgehend zurückgehalten und der durch die Schüttung separierte und durchgesickerte Substrat-Flüssigkeitsanteil wird für eine weitere Verarbeitung aufgefangen. Dazu kann der Separatorboden einen höhenversetzten Gitterboden aufweisen, auf dem die Schüttung aufliegt und unter dem sich der separierte Substrat-Flüssigkeitsanteil sammelt.

Die Schüttung mit dem separierten Substrat-Feststoffanteil wird kontinuierlich oder chargenweise unmittelbar in den Fermenter und/oder über die Zudosiereinrichtung dem Fermenter zudosiert, wobei entsprechend der Entnahme aus dem Separatorbehälter jeweils eine frische Schüttung nachgesteuert wird.

Ein solcher Bio-Separator kann sehr kostengünstig eingerichtet und betrieben werden. Je nach Art der Schüttung, beispielsweise in der Art von Strohlagen der Dichte der Schüttung und der Verweildauer, kann das Separiervermögen und damit der Anteil der separierten Feststoffe beeinflusst und grob eingestellt werden. Weiter kann durch die Verweildauer der Schüttung im Separatorbehälter ein Anmaischgrad und/oder die Einleitung eines Gärprozesses begünstigt durch die im Substrat enthaltenen Bakterien beeinflusst und vorgegeben werden. Um möglicherweise in geringem Umfang bereits entstehendes Biogas aufzufangen, kann der Separatorbehälter auch geschlossen sein.

Im vorstehenden Bio-Separator kann somit ein Teil der für die Zudosierung in den Separator vorgesehenen Biofeststoffe vorerst als Schüttung im Separator eingesetzt werden. Gemäß Anspruch 7 ist es gegebenenfalls vorteilhaft einen solchen Separator zugleich als alleinige Feststoff-Zudosiereinrichtung einzusetzen und die gesamte zudosierbare Biomasse als Schüttung vorerst im Separator zu verwenden und erst anschließend mit einem darin aufgenommenen Substrat-Feststoffanteil dem Fermenter zuzuführen.

Mit Anspruch 8 wird vorgeschlagen, mittels der Zudosiereinrichtung als Feststoffzudosierer dem Fermenter nur Feststoffe zuzuführen, wobei durch den Gärprozess darin gebundene Flüssigkeitsanteile freigesetzt werden. Ein solcher Betrieb ist insbesondere in Verbindung mit einer Trockensubstanzregelung gemäß Anspruch 12 vorteilhaft.

Nach Anspruch 9 können vorteilhaft ausschließlich nachwachsende Rohstoffe (NaWaRos) für eine Fermenterbeschickung verwendet werden. Dadurch ergibt sich bezüglich des CO₂-Kreislaufs eine hohe Umweltverträglichkeit, welche durch staatliche Zuwendungen honoriert wird.

Gemäß Anspruch 10 kann das dem Separator zugeführte Substrat unmittelbar dem ersten Fermenter und/oder einem diesem Fermenter nachgeordneten Nachfermenter entnommen werden, wobei bei einer Zudosierung vorerst über die Anordnung Fermenter-Nachfermenter ein Niveauausgleich erfolgt und gegebenenfalls etwas zeitversetzt Substrat aus dem Nachfermenter abgezogen wird.

In einer Verfahrensführung nach Anspruch 11 wird dem Separator die gesamte aus dem Fermenter oder gegebenenfalls dem Nachfermenter entnommene Substratmenge zugeführt. Aus dem nach dem Separator anfallenden Substrat-Flüssigkeitsanteil wird eine Teilmenge, vorzugsweise im Bereich von 20% der Gesamtflüssigkeitsmenge mittels einer Pumpeinrichtung in den Fermenter für Verdünnungszwecke zurückgeführt. Die verbleibende Restmenge von ca. 80% des Substrat-Flüssigkeitsanteils wird zweckmäßig einem Endlager zugeführt und/oder als Dünger auf Freiflächen ausgebracht.

Weiter ist nach Anspruch 12 als vorteilhafte Weiterbildung eine Trockensubstanzregelung für das Substrat im Fermenter vorgesehen. Dazu wird ein bestimmter Soll-Trockensubstanzgehalt im Substrat des Fermenters vorgegeben, bei dem insbesondere das Substrat für eine Entnahme zum Niveauausgleich auch bei ggf. hohem Trockensubstanzgehalt noch fließ- und pumpfähig ist. Zudem wird der Ist-Trockensubstanzgehalt im Substrat des Fermenters erfasst und bei einer Überschreitung des Soll-Trockensubstanzgehalts durch den erfassten Ist-Trockensubstanzgehalt wird dieser auf den vorgegebenen Soll-Trockensubstanzgehalt durch Flüssigkeitszugabe zurückgeführt, indem die erforderliche Regel-Flüssigkeitsmenge zur Verdünnung in den Fermenter aus dem am Separator anfallenden Substrat-Flüssigkeitsanteil direkt oder indirekt zurückgeführt und eingespeist wird.

Damit wird vorteilhaft eine einstufige, zur Nassfermentation im Durchflussverfahren arbeitende Biogasanlage im eingefahrenen Betriebszustand ohne Zugabe von Fremdflüssigkeit betreibbar. Ohne Zugabe von Fremdflüssigkeit kann eine solche Biogasanlage vorteilhaft in Verbindung mit landwirtschaftlichen Betrieben, die keine Viehhaltung betreiben und damit keine Gülle zur Verfügung haben, eingesetzt werden. Insbesondere ist kein Frischwasser als Fremdflüssigkeit erforderlich, was zur Senkung der Betriebskosten und zur Erhöhung der Umweltfreundlichkeit beiträgt. Zudem kann bei diesem Betrieb der staatliche Technologiebonus für eine weitere Steigerung der Wirtschaftlichkeit in Anspruch genommen werden, der als Aufschlag pro elektrisch erzeugter Energieeinheit ausgezahlt wird.

Eine Automatisierung der Trockensubstanzregelung nach Anspruch 13 mit einer Trockensubstanz-Regeleinrichtung reduziert den für die Biogasanlage erforderlichen Arbeitsaufwand. Die Trockensubstanz-Regelung kann dabei ggf. in Verbindung mit weiteren erforderlichen Regelungen in entsprechenden Geräten und Schrankanordnungen integriert werden. Durch die Möglichkeit der Vorgabe eines Soll-Trockensubstanzgehalts kann dieser auf die jeweiligen Gegebenheiten optimiert werden, insbesondere zur Optimierung der Vergärung, für ein weitgehend gleiches Fermenterfüllniveau und zur Gewährleistung der Fließfähigkeit und Pumpfähigkeit des Substrats im Fermenter.

Nach Anspruch 14 wird je nach der konkreten Ausführung einer Biogasanlage die in den Fermenter zurückgeführte Teilmenge des separierten Substrat-Flüssigkeitsanteils gegebenenfalls als Regel-Flüssigkeitsmenge unmittelbar in den Fermenter zurückgeführt und/oder mittelbar zumindest teilweise über eine Vorgrube als Vorratsgefäß und/oder als Anmaischgrube zurückgeführt.

Anhand einer Zeichnung wird die Erfindung näher erläutert.

Es zeigen:
- Fig. 1: eine schematische Draufsicht auf eine Biogasanlage,
- Fig. 2: eine Biogasanlage ähnlich Fig. 1 mit einem speziellen Separator, dieser schematisch in einer Seitenansicht, und
- Fig. 3: eine schematische Draufsicht auf eine weitere modifizierte Biogasanlage.

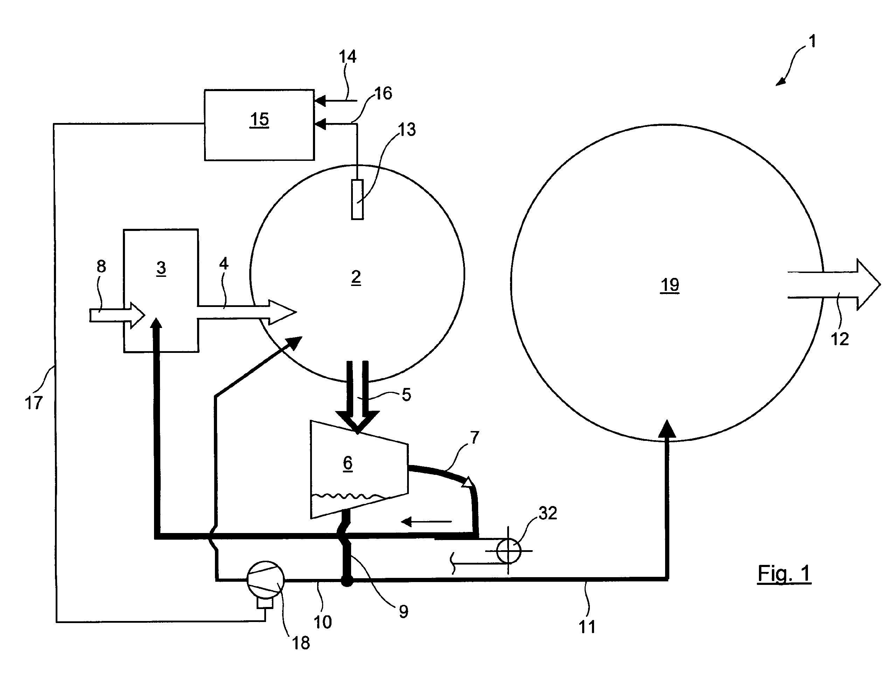

In Fig. 1 ist eine Biogasanlage 1 in einer schematischen Draufsicht gezeigt mit einem Fermenter 2 und einem Endlager 19. Dem Fermenter 2 ist eine Zudosiereinrichtung 3 vorgeschaltet, mit der kontinuierlich oder chargenweise quasi kontinuierlich vergärbare Stoffe dem Fermenter 2 über (nicht im einzelnen dargestellte) Fördermittel 4 zugeführt werden.

Zur Stabilisierung des Flüssigkeitsniveaus im Fermenter 2 wird von dort eine der Zudosierung entsprechende Menge an Substrat durch Verdrängen oder Pumpen über eine (schematisch angedeutete) Förderleitung 5 entnommen und insgesamt einem Separator 6, beispielsweise einem Schraubenseparator oder einer Siebbandpresse zugeführt.

Das über die Förderleitung 5 abgezogene Substrat wird in einer Fest-Flüssig-Trennung im Separator 6 separiert zu einem Substrat-Feststoffanteil und einem Substrat-Flüssigkeitsanteil. Der Substrat-Feststoffanteil wird in der schematischen Darstellung vom Separator 6 ausgeworfen (Pfeil 7) auf ein Fördermittel, hier schematische als Teil eines Förderbands 32 dargestellt. Damit wird der gesamte separierte Substrat-Feststoffanteil der Zudosiervorrichtung 3 wieder zugeführt und dort zusammen mit zugegebenen vergärbaren Stoffen (Pfeil 8), insbesondere nachwachsenden Rohstoffen (NaWaRos) für eine erneute Beschickung in den Fermenter 2 bereitgehalten. Der separierte Substrat-Flüssigkeitsanteil wird im oder nach dem Separator 6 aufgefangen und als Efluent über die Flüssigkeitsleitung 9 abgeführt. Diese verzweigt sich in eine Flüssigkeitsleitung 10, welche über eine Dosierpumpe 18 für eine teilweise Wiedereinspeisung des Substrat-Flüssigkeitsanteils zum Fermenter 2 führt und in eine Flüssigkeitsleitung 11 mit dem der nicht dem Fermenter 2 zugeführte Substrat-Flüssigkeitsanteil in das Endlager 3 gelangt. Von dort kann das gelagerte Efluent insbesondere als Flüssigdünger ausgebracht werden (Pfeil 12). Vorteilhaft sind in diesem Flüssigdünger wenig Feststoffanteile enthalten, so dass ein sonst ungünstiges Anhaften mit einem Verkleben und Verschließen von Poren und Kapillaren an Pflanzen weitgehend vermieden wird.

Zudem ist im Fermenter 2 eine schematisch dargestellte Sonde 13 zur Erfassung eines Ist-Trockensubstanzgehalts eingebracht, deren Messwert als Istwert (Pfeil 16) einem Regler 15 zugeführt ist, an dem zudem ein Soll-Trockensubstanzgehalt (Pfeil 14) vorgebbar ist. Bei einer Regelabweichung wird vom Regler 15 über eine Steuerleitung 17 ein bewertetes Stellsignal für eine ermittelte Mengenzudosierung eines Substrat-Flüssiganteils an die Dosierpumpe 18 abgegeben. Dadurch kann gegebenenfalls auf eine Zudosierung von Fremdflüssigkeit verzichtet werden.

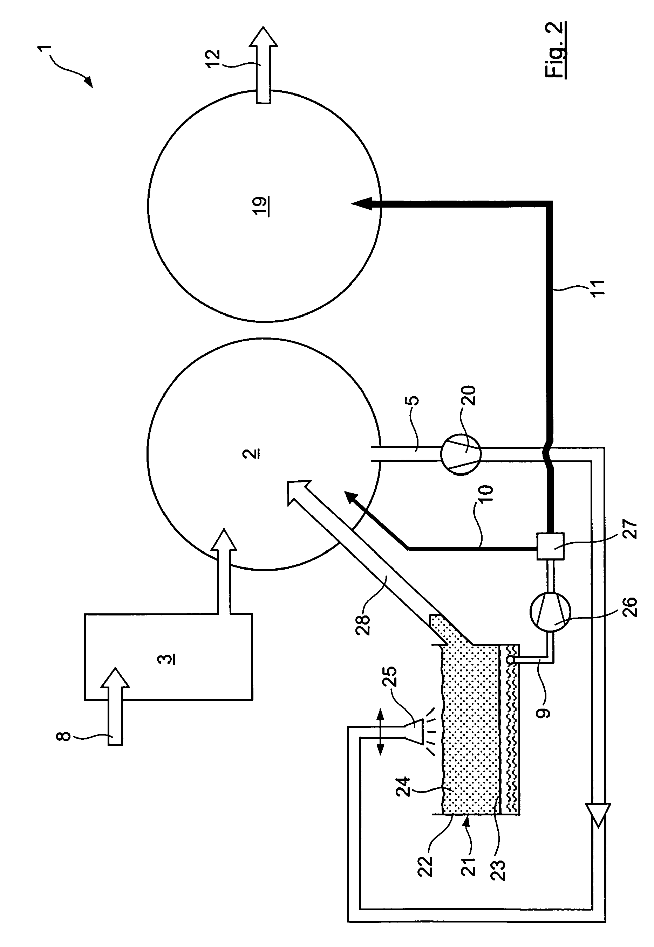

Fig. 2 zeigt eine ähnliche Biogasanlage 1, jedoch ohne Regeleinrichtung und mit einem speziellen Bio-Separator, der in einer schematischen Seitenansicht dargestellt ist. Für gleiche Teile sind gleiche Bezugszeichen verwendet.

Auch in der Biogasanlage 1 nach Fig. 2 sind ein Fermenter 2, eine Zudosiereinrichtung 3 und ein Endlager 19 vorgesehen. Vom Fermenter 2 wird wie in der Ausführung nach Fig. 1 bei einer Zudosierung über die Förderleitung 5 eine entsprechende Menge an Substrat entnommen und hier mit einer Pumpe 20 einer speziellen Ausführung eines Separators, hier als Bio-Separator 21 bezeichnet, zugeführt. Beim Bio-Separator 21 ist in einen wannenartigen Separatorbehälter 22 auf einem Gitterzwischenboden 23 eine Schüttung 24 aus Biofeststoffen, beispielsweise eine Strohschüttung, eingebracht. Über diese wird durch einen gegebenenfalls in der Fläche bewegbaren Ausströmer 25 das entnommene Substrat geleitet. Dadurch wird in der Schüttung 24 der Substrat-Feststoffanteil aus dem aufgebrachten Substrat weitgehend zurückgehalten. Unterhalb des Gitterzwischenbodens 23 sammelt sich der durchsickernde separierte Substrat-Flüssigkeitsanteil und wird über die Flüssigkeitsleitung 9 durch die Pumpe 26 weggepumpt. Nach der Pumpe 26 ist in der Leitung ein 3-Wege-Ventil 27 eingesetzt, so dass je nach dessen Stellung ein bestimmter Substrat-Flüssigkeitsanteil über die Flüssigkeitsleitung 10 dem Fermenter 2 oder über die Flüssigkeitsleitung 11 dem Endlager 19 zugeführt werden kann.

Aus dem Bio-Separator 21 wird die mit Substrat-Feststoffanteilen angereicherte Schüttung entweder direkt (schematisch dargestellter Transportweg 28) oder über einen (nicht dargestellten) Transportweg der Zudosiervorrichtung 3, ähnlich wie in Fig. 1, zugeführt. Wenn die gesamte Beschickungsmenge zuerst als Schüttung im Bio-Separator 21 eingesetzt wird, kann dieser gegebenenfalls als alleinige Zudosiereinrichtung aufgebaut und eingesetzt werden, so dass die eingezeichnete Zudosiereinrichtung 3 entfallen kann.

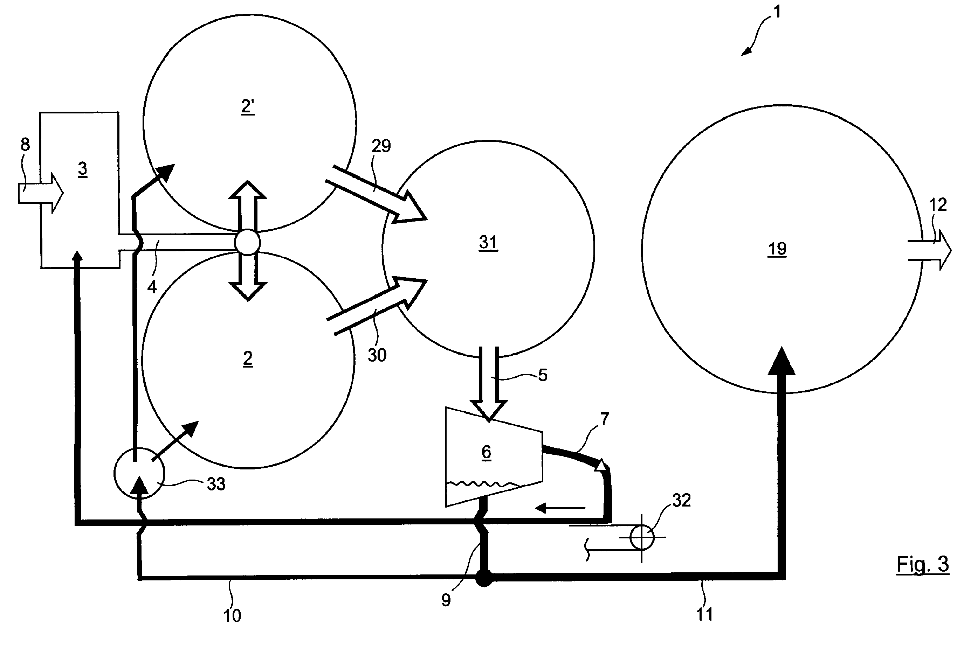

Die Biogasanlage 1 nach Fig. 3 ist eine Modifikation der Ausführungsform nach Fig. 1, wobei auch hier gleiche Bestandteile mit gleichen Bezugszeichen versehen sind. Die wesentlichen Unterschiede bestehen darin, dass zwei Fermenter 2, 2' parallel betrieben werden, welche über die Zudosiereinrichtung 3 und die Fördermittel 4 wechselweise beschickt werden. Aus den Fermentern 2, 2' wird bei der Beschickung das entsprechende Substrat über Förderleitungen 29, 30 nicht direkt dem Separator 6, sondern einem Nachfermenter 31 zugeführt. Aus diesem wiederum erfolgt die entsprechende Substratzuführung über die Förderleitung 5 an den Separator 6.

Von diesem wird der separierte Substrat-Feststoffanteil (Pfeil 7) über ein Förderband 32 der Zudosiereinrichtung 3 zugeführt. Über die Flüssigkeitsleitung 9 und die Verzweigung zu den Flüssigkeitsleitungen 10 und 11 wird hier mittels (nicht dargestellter) Dosierpumpen und/oder Ventilsteuerungen ein jeweils geeigneter Anteil des separierten Substrat-Flüssigkeitsanteils den Fermentern 2, 2' und/oder dem Endlager 19 zugeführt. Die Zuführung zu den Fermentern 2, 2' erfolgt hier in einer weiteren Ausgestaltung über eine Anmaischgrube 33.

Durch die dargestellte Rückführung von Substrat-Feststoffanteilen unmittelbar oder über eine Zudosiervorrichtung in den oder die Fermenter 2, 2' wird im abgezogenen Substrat noch enthaltenes Energiepotential für einen wirtschaftlicheren Betrieb der Biogasanlage genutzt.

## Patentansprüche

1. Betriebsverfahren zur Nassfermentation in einer einstufigen, eingefahrenen und im Durchflussverfahren arbeitenden Biogasanlage (1),
mit wenigstens einem ein Substrat mit bestimmtem Trockensubstanzgehalt enthaltenden Fermenter (2, 2') mit einer vorgeschalteten Zudosiereinrichtung (3) für vergärbare Stoffe und mit einem nachgeordneten Separator (6; 21), mit dem eine zumindest teilweise Trennung eines aus dem Fermenter (2, 2') abgezogenen Substrats in einen Substrat-Feststoffanteil (7) und einen Substrat-Flüssigkeitsanteil (11) erfolgt,
**dadurch gekennzeichnet,**
**dass** mit der Zudosiereinrichtung (3) eine vorgegebene Menge an vergärbaren Stoffen kontinuierlich oder chargenweise quasi kontinuierlich in den Fermenter (2, 2') eingebracht wird und eine entsprechende Menge an Substrat dem Fermenter (2, 2') durch Verdrängung oder Pumpen entnommen und zumindest teilweise dem Separator (6; 21) zugeführt wird, und
**dass** zumindest ein Teil des durch den Separator (6; 21) separierten Substrat-Feststoffanteils (7) unmittelbar in den Fermenter (2, 2') und/oder über die Zudosiereinrichtung (3) in den Fermenter zurückgeführt wird.

2. Betriebsverfahren nach Anspruch 1, **dadurch gekennzeichnet, dass** das gesamte entnommene Substrat dem Separator (6; 21) zugeführt wird.

3. Betriebsverfahren nach Anspruch 1 oder 2, **dadurch gekennzeichnet, dass** der gesamte durch den Separator (6; 21) separierte Substrat-Feststoffanteil (7) in den Fermenter (2, 2') zurückgeführt wird.

4. Betriebsverfahren nach einem der Ansprüche 1 bis 3, **dadurch gekennzeichnet, dass** der Substrat-Feststoffanteil (7) vom Separator (6; 21) automatisiert durch Fördermittel (32) kontinuierlich oder chargenweise der Zudosiereinrichtung (3) zugeführt wird.

5. Betriebsverfahren nach einem der Ansprüche 1 bis 4, **dadurch gekennzeichnet, dass** als Separator (6) ein Schrauben- bzw. Schneckenseparator und/oder ein Zentrifugenseparator und/oder eine Siebbandpresse eingesetzt wird.

6. Betriebsverfahren nach einem der Ansprüche 1 bis 4, **dadurch gekennzeichnet, dass** der Separator (21) einen Separatorbehälter (22) aufweist, mit einer Schüttung (24) aus Biofeststoffen als Feststofffilter, über die das abgezogene Substrat geleitet wird, wobei in der Schüttung (24) der Substrat-Feststoffanteil weitgehend zurückgehalten wird und unter der der **dadurch** separierte Substrat-Flüssigkeitsanteil für die weitere Verarbeitung aufgefangen wird, und
dass die Schüttung (24) mit dem Substrat-Feststoffanteil kontinuierlich oder chargenweise unmittelbar in den Fermenter (2) und/oder über die Zudosiereinrichtung (3) dem Fermenter (2) zudosiert wird mit entsprechender Nachsteuerung einer frischen Schüttung (24).

7. Betriebsverfahren nach Anspruch 6, **dadurch gekennzeichnet, dass** der Separator (21) zugleich zu seiner Separatorfunktion die alleinige Feststoffzudosiereinrichtung ist und die gesamte zudosierbare Biomasse zuerst als Schüttung (24) verwendet wird.

8. Betriebsverfahren nach einem der Ansprüche 1 bis 7, **dadurch gekennzeichnet, dass** mit der Zudosiereinrichtung (3) als Feststoffzudosierer nur Feststoffe zudosiert werden, wobei durch den Gärprozess darin gebundene Flüssigkeitsanteile freigesetzt werden.

9. Betriebsverfahren nach einem der Ansprüche 1 bis 8, **dadurch gekennzeichnet, dass** als vergärbare Stoffe ausschließlich nachwachsende Rohstoffe (NaWaRos) zudosiert werden.

10. Betriebsverfahren nach einem der Ansprüche 1 bis 9, **dadurch gekennzeichnet, dass** aus dem der Zudosiereinrichtung (3) unmittelbar nachgeordneten Fermenter (2, 2') und/oder einem diesem Fermenter (2, 2') nachgeordneten Nachfermenter (31) das Substrat für den Separator (6) abgezogen wird.

11. Betriebsverfahren nach Anspruch 10, **dadurch gekennzeichnet, dass** die der zudosierten Stoffmenge entsprechende, dem Fermenter (2, 2') und/oder ggf. dem Nachfermenter (31) entnommene Substratmenge insgesamt dem Separator (6; 21) zugeführt wird und von dem separierten Substrat-Flüssiganteil eine Teilmenge, vorzugsweise im Bereich von 20% der Gesamtflüssigkeitsmenge mittels einer Pumpeinrichtung (18) oder Ventilanordnung (27) in den Fermenter (2, 2') zurückgeführt wird, und
dass die Restmenge von etwa 80% des Substrat-Flüssigkeitsanteils in einen Nachfermenter (31) oder in ein Endlager (19) überführt und/oder auf Freiflächen ausgebracht wird.

12. Betriebsverfahren nach einem der Ansprüche 1 bis 11, **dadurch gekennzeichnet, dass** eine Trockensubstanzregelung für das im Fermenter befindliche Substrat vorgesehen ist dergestalt,
dass ein bestimmter Soll-Trockensubstanzgehalt im Substrat des Fermenters (2, 2') vorgegeben wird, bei dem insbesondere das Substrat auch bei gegebenenfalls hohem Trockensubstanzgehalt noch fließ- und pumpfähig ist,
dass der Ist-Trockensubstanzgehalt im Substrat des Fermenters (2, 2') erfasst wird,
dass bei einer Überschreitung des Soll-Trockensubstanzgehalts durch den erfassten Ist-Trockensubstratgehalt dieser auf den Soll-Trockensubstanzgehalt zurückgeführt wird, indem die erforderliche Regel-Flüssigkeitsmenge zur Verdünnung in den Fermenter (2, 2') aus dem am Separator (6; 21) anfallenden Substrat-Flüssigkeitsanteil direkt oder indirekt zurückgeführt und eingespeist wird, so dass keine Zugabe zusätzlicher Fremdflüssigkeit erfolgt.

13. Betriebsverfahren nach Anspruch 12, **dadurch gekennzeichnet, dass** in einer Trockensubstanz-Regeleinrichtung der Ist-Trokkensubstanzgehalt im Substrat des Fermenters (2, 2') über eine Sonde (13) gemessen wird und der Messwert als elektrisches Ist-Signal einem Regler (15) zugeführt wird, an dem zudem der Soll-Trockensubstanzgehalt vorzugsweise zwischen 10% und 15,5% über ein elektrisches Soll-Signal einstellbar ist, und
dass vom Regler (15) nach Bewertung einer Regelabweichung über einen Regelalgorithmus ein Stellsignal an ein Regelstellglied (Dosierpumpe 18) zur Bereitstellung und Zuführung der Regel-Flüssigkeitsmenge abgegeben wird.

14. Betriebsverfahren nach einem der Ansprüche 11 bis 13, **dadurch gekennzeichnet, dass** die in den Fermenter (2, 2') zurückgeführte Teilmenge des separierten Substrat-Flüssigkeitsanteils, gegebenenfalls als Regel-Flüssigkeitsmenge unmittelbar in den Fermenter (2, 2') zurückgeführt und/oder mittelbar zumindest teilweise über eine Vorgrube als Vorratsgefäß und/oder als Anmaischgrube (33) zurückgeführt wird.
